# EUROPÄISCHE PATENTSCHRIFT

(11) **EP 1 185 510 B1**
(45) Veröffentlichungstag und Bekanntmachung des Hinweises auf die Patenterteilung: **03.03.2004**
(21) Anmeldenummer: 00934977.0
(22) Anmeldetag: 02.05.2000
(51) Int. Cl.: C07C 323/58, C07C 323/59, C07C 329/06, A61P 9/08

(54) **S-NITROSO- UND S-NITRO-N-ACYL-L-CYSTEIN-ESTER-DERIVATE ALS PHARMAKOLOGISCHE WIRKSTOFFE UND DIESE VERBINDUNGEN ENTHALTENDE ARZNEIMITTEL**
S-NITROSO- AND S-NITRO-N-ACYL-L-CYSTEINE-ESTER-DERIVATIVES AS PHARMACOLOGICAL SUBSTANCES AND MEDICAMENTS CONTAINING SAID COMPOUNDS
DERIVES D'ESTERS DE S-NITROSO- ET S-NITRO-N-ACYL-L-CYSTEINE EN TANT QUE PRINCIPES ACTIFS PHARMACOLOGIQUES ET MEDICAMENTS CONTENANT CES COMPOSES

(30) Priorität: 30.04.1999 DE 19919941
(43) Veröffentlichungstag der Anmeldung: 13.03.2002
(73) Patentinhaber: Tsikas, Dimitrios, 30974 Wennigsen (DE)
(72) Erfinder: Tsikas, Dimitrios, 30974 Wennigsen (DE)
(74) Vertreter: VOSSIUS & PARTNER
(86) Internationale Anmeldenummer: PCT/EP2000/003934
(87) Internationale Veröffentlichungsnummer: WO 2001/010828

(56) Entgegenhaltungen:
- US-A- 5 187 305
- BONNETT, R. & NICOLAIDOU, P.: "Nitrosation and Nitrosylation of Haemoproteins and Related Compounds. Part 2. The Reaction of Nitrous Acid with the Side Chains of alpha-Acyl-amino-acid Esters" JOURNAL OF THE CHEMICAL SOCIETY - PERKIN TRANSACTIONS I, Bd. 1979, Nr. 8, 1979, Seiten 1969-1977, XP000940554
- PETIT, C. ET AL.: "Novel donors of nitric oxide derived of S-nitrosocysteine possessing antioxidant activities" BRAZILIAN JOURNAL OF MEDICAL AND BIOLOGICAL RESEARCH, Bd. 32, Nr. 11, November 1999 (1999-11), Seiten 1407-1412, XP000918769 & 10-13/03/1999: , PROCEEDINGS OF THE MEETING "NO BRAZIL, BASIC AND CLINICAL ASPECTS OF NITRIC OXIDE"

## Beschreibung

Die Erfindung betrifft neue S-Nitroso- und S-Nitro-N-Acyl-L-Cystein-Ester-Derivate (SNACE-Derivate) als pharmakologische Wirkstoffe und Arzneimittel - insbesondere zur transdermalen Applikationen -, die ein SNACE-Derivat enthalten, zur Prophylaxe und Behandlung einer Reihe von Krankheiten, einschließlich peripherer Verschlußkrankheiten, des Myokardinfarktes, der Angina pectoris, des ischämischen Schlaganfalls, arteriosklerotischer Gefäßveränderungen, der Thrombose (Hemmung der Plättchenaggregation), der Homocysteinämie, sowie der Impotentia coeundi, der Miktionsstörung, des Asthma bronchiale, der Hypertonie, der pulmonalen Hypertonie, der diabetischen Nephropathie, der Mukoviszidose, von Krebs und der Alzheimer-Krankheit.

Eine Vielzahl von Vasodilatatoren sind bekannt, die zur Behandlung von hypertensiven Zuständen, der Angina pectoris, der Impotentia coeundi, der Thrombose und von anderen Krankheiten verwendet werden können. Diese Arzneimittel können nach dem Mechanismus ihrer primären Wirkung in verschiedenen Kategorien eingeteilt werden. Drei wichtige Gruppen dieser Arzneimittel sind die Inhibitoren des Angiotensin Converting Enzyme (ACE), die organischen Nitrate und Stickstoffmonoxid (NO)-Donoren. Während weder ACE-Inhibitoren noch die organischen Nitrate wie z.B. Glyceroltrinitrat (GTN) klinisch bedeutsame antiaggregatorische Wirkstoffe sind, können einige NO-Donoren einschließlich der S-Nitroso-Substanzen (RSNO) sowohl antihypertensiv als auch antiaggregatorisch wirken.

Im Gegensatz zu den organischen Nitraten, welche ihre Wirkung erst nach ihrer Metabolisierung in den Zellen zu aktiven Intermediaten entfalten, wirken S-Nitroso-Verbindungen ohne vorherige enzymatische Aktivierung entweder direkt oder nach Freisetzung von NO. Ignarro et al. (Biochem. Biophys. Acta, 631 (1980) 221-231) haben gezeigt, daß die S-Nitroso-Verbindungen von Cystein, Glutathion, Penicillamin und Dithiothreitol in der Lage sind, die lösliche Guanylat-Cyclase (sGC) direkt zu aktivieren. Möglicherweise beruht die Wirkung von organischen Nitraten, von Natriumnitrit und Nitroprussidnatrium auf der Aktivierung der sGC nach intermediärer Bildung von S-Nitroso-Substanzen aus diesen Substanzen und den Thiolen der Zellen. Die Abhängigkeit der Wirkung von organischen Nitraten von ihrem Metabolismus und dem Thiol-Status einer Zelle könnte die Toleranzentwicklung gegen organische Nitrate erklären. Es wurde gezeigt, daß S-Nitroso-Substanzen wie z.B. S-Nitroso-N-acetylpenicillamine (SNAP) in vivo am Tier keine Toleranzentwicklung zeigen (Shaffer JE et al. J. Pharmacol. Exper. Ther. 260 (1992) 286-293). Es ist dokumentiert, daß S-Nitroso-Substanzen wie z B. S-Nitrosoglutathion in vitro und in vivo am Menschen antihypertensive and antiaggregatorische Wirkungen haben (De Belder et al., Cardiovasc. Res., 28 (1994) 691-694). Fur S-Nitrosoglutathion wurde sogar gezeigt, daß seine antiaggregatorische Komponente sehr viel stärker ausgepragt ist als die antihypertensive (De Belder et al., Cardiovasc. Res, 28 (1994) 691-694). Für S-Nitrosoglutathion wurde auch gezeigt, daß es am Menschen die Embolierate reduziert (Molloy et al., Circulation, 98 (1998) 1372-1375) und daß diese Wirkung über die von Acetylsalicylsäure und Heparin hinausgeht.

Die Mechanismen der antiaggregatorischen Wirkung von S-Nitroso-Substanzen sind bis heute noch nicht vollständig geklärt. Neben dem cGMP-abhangigen Mechanismus werden verschiedene cGMP-unabhängige Mechanismen diskutiert, die auf der Beeinflussung von Enzymaktivitäten bzw. Rezeptoren durch S-Nitroso-Substanzen basieren. Es wurde beispielsweise gezeigt, daß verschiedene S-Nitroso-Substanzen und NO die Enzyme Glyceraldehyd-3-Phosphate-Dehydrogenase (Padgett und Whorton, Am. J. Physiol. 269 (Cell Physiol. 38), (1995) C739-C749), Phosphodiesterase (Maurice und Haslam RJ, Molecular Pharmacol., 37 (1990) 671-681) und Cyclooxyeenase hemmen (Tsikas D et al., FEBS Lett., 442 (1999) 162-166).

Die Verwendung von S-Nitroso-Derivaten von ACE-Inhibitoren und Cystein zur Behandlung von verschiedenen Krankheiten wurde von Loscalzo J und Cooke J (US Patent 5,025,001, 1991) sowie Stamler JS und Loscalzo J (International Application Number PCT/US92/03008) beschrieben.

Bonnet, R. Nicolaidoun, P., J. Chem. Soc. Perkin Trans. I 1979, S. 1969-1974 offenbart S-Nitroso-N-Acetylcysteinmethylester.

Petit C. et al., Braz. J. Med. Biol. Res., 32(11) 1999, S. 1407-1412 und US-A-5,187,305 offenbaren S-Nitroso-N-Acetylcysteinesterderivate als NO-Donatoren.

Zur Zeit sind keine S-Nitroso- und S-Nitro-Substanzen als Medikamente zugelassen.

Der Erfindung liegt die Aufgabe zugrunde, SNACE-Derivate als Arzneistoffe bzw pharmakologische Wirkstoffe zur Verfügung zu stellen. Die erfindungsgemäßen SNACE Derivate eignen sich insbesondere für Arzneimittel zur transdermalen Applikation, da sie durch die Haut resorbiert, und nach Resorption entweder selbst oder als Metabolite (z.B. von Hydrolasen, Deacylasen) direkt oder indirekt als "Pro-Drug" ihre Wirkung entfalten.

Gegenstand der Erfindung sind somit *S*-Nitroso- und *S*-Nitro-*N*-Acyl-L-Cystein-Ester-Derivate der allgemeinen Formel I: 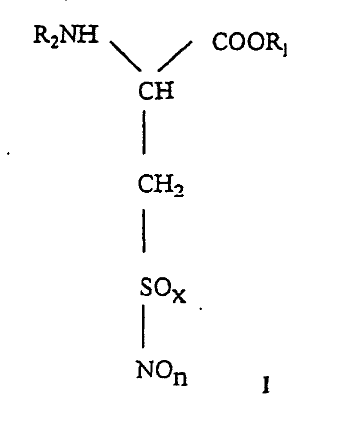 in der n = 1 oder n = 2 ist, x = 0, 1 oder 2 ist, R₁ ein Alkylrest (C₁₋₂₂) ist und R₂ ein Acylrest (C₂₋₂₂) oder *O*-Acetylsalicylrest oder ein Alkylrest (C₁₋₂₂) ist, als pharmakologische Wirkstoffe mit Ausnahme von S-Nitroso-N-acetylcysteinethylester.

Bevorzugt sind die S-Nitroso- (d.h. n = 1 und x = 0) und S-Nitro-Gruppen (d.h. n = 2 und x = 0).

Bevorzugt sind Alkyl- und Acylreste mit bis zu 6 Kohlenstoffatomen.

Beispiele für Alkylreste sind die Methyl-, Ethyl-, n-Propyl-, und tert.-Butylgruppe.

Beispiele für Acylreste sind die Acetyl-, Priopionyl-, und tert.-Butyryl- und *O*-Acetylsalicyl-Gruppe.

Die besonders bevorzugten Alkyl- und Acylreste sind die Ethyl-, Acetyl- und *O*-Acetylsalicylgruppe wegen der Ungiftigkeit ihrer Abspaltprodukte Ethanol, Essigsäure und *O*-Acetylsalicylsäure oder Salicylsäure.

Die pharmakologisch wirksame Gruppe der Verbindungen der allgemeinen Formel I ist die S-Nitroso-Gruppe (-S-N=O) bzw. S-Nitro-Gruppe (S-NO₂). Die Alkyl- und Acyl-Reste und die Substituenten am Schwefel der Verbindungen der allgemeinen Formel I beeinflussen in erster Linie die physikochemischen Eigenschaften, z.B. die Penetrationsrate in der Haut, und weniger ihre pharmakologische Aktivität.

Die Herstellung der Verbindungen der allgemeinen Formel I erfolgt nach an sich bekannten Methoden durch Veresterung der Carboxylgruppe von Cystein und *N*-Acylierung bzw. *N*-Alkylierung der Aminogruppe von Cystein. Ester werden z.B. durch Verwendung von 3 M HCl in den entsprechenden wasserfreien Alkoholen hergestellt. *N*-Acyl-Derivate werden z.B. durch Verwendung von Anhydriden der entsprechenden Carbonsäuren hergestellt. *N*-Alkyl-Derivate werden z.B. durch Verwendung von Alkylhalogeniden hergestellt. Die Verbindungen der allgemeinen Formel II sind die Vorstufen der entsprechenden Verbindungen mit der allgemeinen Formel I. Die Nitrosylierung der Thiolgruppe von Cystein-Derivaten der allgemeinen Formel II erfolgt in Salzsäure-saurer Lösung durch stöchiometrische Mengen von Natriumnitrit (Tsikas et al., Anal. Biochem. 244 (1997) 208-220). Die Nitrierung der Thiolgruppe von Verbindungen der allgemeinen Formel II erfolgt z.B. durch Verwendung von Nitroniumtetrafluoroborat (Balazy et al., J. Biol. Chem. 273 (1998) 32009-32015).

Alternativ erfolgt die Herstellung der Verbindungen der allgemeinen Formel 1 (n = 1 oder 2 und x = 0) durch S-Transnitrosylierung bzw. S-Transnitrierung der. Thiolgruppe der entsprechen Cystein-Derivate der allgemeinen Formel 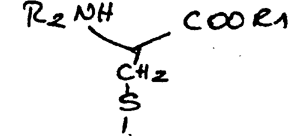 mit Hilfe von z.B. S-Nitrosoglutathion bzw. S-Nitroglutathion (Tsikas et al., Anal. Biochem. 270 (1999) 231-241). Diese Methode kann auch für die in situ-Herstellung der Verbindungen der allgemeinen Formel I im Arzneimittel angewendet werden. Die SNACE-Derivate mit x = 1 und x = 2 werden aus den entsprechenden Derivaten mit x = 0 durch Oxidation z.B. Wasserstoffperoxid hergestellt.

Die Verbindungen mit der allgemeinen Formel 1 können zur Prävention der Arteriosklerose, zur Behandlung der Hypertonie und pulmonalen Hypertonie, zur Prävention und Behandlung von Durchblutungsstörungen, z.B. des Hirns, Herzens und der Extremitäten, zur Hemmung der Plättchenaggregation (Thrombozyten-Aggregation), zur Behandlung der Impotentia coeundi und zur Behandlung von Asthma bronchiale, zur Behandlung der Cystischen Fibrose (Mukoviszidose) und von Abstoßungsreaktionen bei Transplantationen sowie von Krebs, Homocysteinämie und zur Lipidsenkung verwendet werden. Typische Beispiele für Störungen der Hirndurchblutung sind transitorische zerebrale Ischämie, Hörsturz, Schwindelzustände auf der Basis von Durchblutungsstörungen und ischämischer Schlaganfall. Typische Beispiele für Störungen der Herzdurchblutung sind Angina pectoris und Herzinfarkt. Typische Beispiele für Störungen der Durchblutung der Extremitäten sind periphere arterielle Durchblutungsstörungen bei Arteriosklerose, M. Burgers und M. Raynaud sowie Raynaud-Syndrom.

Neben der transkutanen kommen für die Verbindungen der allgemeinen Formel I auch die orale, rektale, intravenöse oder intraarterielle sowie die inhalative Applikationen jeweils auch in Liposomen, Mikroemulsionen und Mikrokapseln in Betracht.

Die Herstellung von Arzneimitteln erfolgt nach üblichen Methoden. Zur Herstellung von Arzneimitteln zur transkutanen Applikation können die Verbindungen der allgemeinen Formel I in der Abwesenheit oder Anwesenheit der entsprechenden Verbindungen der allgemeinen Formel 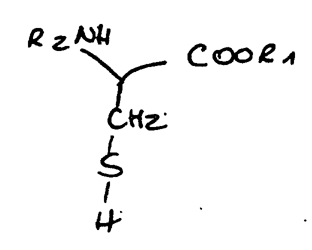 in einer Gelgrundlage, Salbengrundlage bzw. flüssigen Grundlage ohne oder mit verschiedenen Lösungsvermittlern, Penetrationsbeschleunigern sowie Stabilisatoren eingebrächt werden. Als primäre Packmittel werden Sprays, Tuben, Ampullen sowie Einzelportionen verwendet. Nach dem Auftragen auf die Haut oder mit zusätzlichem Okklusionsverband wird der Wirkstoff resorbiert.

Die Verbindungen mit der allgemeinen Formel 1 können gegebenenfalls auch mit Stabilisatoren sowie Lösungsvermittlern auf ein Pflaster aufgebracht und appliziert werden.

### BEISPIEL 1

### A. Herstellung of S-Nitroso-N-acetylcysteinethylester

### I. Herstellung der Vorstufe N-Acetylcysteinethylester (NACET).

L-Cysteineethylester-hydrochlorid (9.2 g, 50 mmol) wird in Methylenchlorid (100 ml) suspendiert. Die Suspension wird mit gesättigter NaHCO₃ (25 ml) und mit 2 M NaOH (20 ml) alkalisiert. Nach der Phasentrennung wird die organische Phase dekantiert und die verbleibende wäßrige Phase mit Methylenchlorid (30 ml) extrahiert. Die organischen Phasen werden kombiniert, über wasserfreiem Na₂SO₄ getrocknet und filtriert. Die klare Lösung wird mit Essigsäureanhydrid versetzt (insgesamt: 4 g, 39.2 mmol) bis zur Vollständigkeit (kontrolliert mit Dünnschichtchromatographie: Silica, Diethylether:Methanol, 9/1, v/v, unter Verwendung von Cerammoniumnitrat zur Detektion). Das Lösungsmittel wird zur Trockene eingeengt und der Rückstand auf Silica mit Diethylether zur Elution chromatographiert. Alle Operationen werden bei Raumtemperatur unter einer Argon-Atmosphäre durchgeführt. Weisse Kristalle (6.2 g, Schmelzpunkt: 44.1-44.5 °C) werden erhalten. Die Verbindung wird mittels Massenspektrometrie, ¹H-NMR, IR und Polarimetrie als N-Acetylcysteineethylester identifiziert (C₇H₁₃NO₃S, MG 191).

### II. Herstellung von S-Nitroso-N-acetylcysteinethylester (SNACET).

Es wird ein Gemisch aus N-Acetylcysteinethylester (9.55 mg, 50 µmol) und Natriumnitrit (3.45 mg, 50 µmol) in deoxygeniertem, distilliertem, eiskaltem Wasser hergestellt. Diese Lösung wird mit einer deoxygenierten, eiskalten 5 M HCl-Lösung (12 µl, 60 µmol) versetzt. Unmittelbar nach der Ansäuerung wird die Lösung rot. Die entstandene Substanz wurde mittels MS, ¹H-NMR, IR und Spektrophotometrie (Molarabsorptionskoefficient von 780 M-¹cm⁻¹ bei einer Wellenlänge von 338 nm der maximalen Absorption) als S-Nitroso-N-acetylcysteinethylester (C₇H₁₂N₂O₄S, MG 220) identifiziert. Die Reinheit der Substanz wird mittels Umkehrphase-Hochdruckflüssigkeitschromatographie (HPLC, Detektion bei 338 nm) zu >99 % bestimmt.

### B. Herstellung von S-Nitroso-N-(O-acetylsalicyl)cysteinethylester

### I. Herstellung der Vorstufe N-(O-Acetylsalicyl)cysteinethylester (ASS-CET).

Cysteinethylester-hydrochlorid (925 mg, 5 mmol) wird in Tetrahydrofuran (30 ml) unter Eiskühlung 2 h lang mit Triethylamin (510 mg) versetzt. Die entstandene Suspension wird mit einer Lösung von Acetylsalicylsäureanhydrid (1.71 g, 5 mmol) in Tetrahydrofuran (10 ml) versetzt und das Gemisch wird 2 h lang gerührt. Das Reaktionsgemisch wird mit Wasser verdünnt und die Reaktionsprodukte werden mit Diethylether extrahiert. Der Extrakt wird anschließend mit NaHCO₃ gewaschen und über wasserfreiem MgSO₄ getrocknet. Das Lösungsmittel wird zur Trockene eingeengt und der Rückstand auf Silica mit Diethylether/Petrolether (1/1, v/v) zur Elution chromatographiert. Alle Operationen werden bei Raumtemperatur unter einer Argon-Atmosphäre durchgeführt. Weisse Kristalle (950 mg, Schmelzpunkt: 76-77 °C) werden erhalten. Die Verbindung wird mittels Massenspektrometrie, ¹H-NMR, IR und Polarimetrie als N-(*O*-acetylsalicyl)cysteineethylester identifiziert (C₁₄H₁₇NO₅S, MG 311).

### II. Herstellung von S-Nitroso-N-(O-acetylsalicyl)cysteinethylester (ASS-SNCET).

Es wird ein Gemisch aus N-(*O*-acetylsalicyl)cysteineethylester (15.6 mg, 50 µmol) und Natriumnitrit (3.45 mg, 50 µmol) in deoxygeniertem, destilliertem, eiskaltem Wasser hergestellt. Diese Lösung wird mit einer deoxygenierten, eiskalten 5 M HCl-Lösung (12 µl, 60 µmol) versetzt. Unmittelbar nach der Ansäuerung wird die Lösung rot. Die entstandene Substanz wurde mittels MS, ¹H-NMR, IR und Spektrophotometrie (Molarabsorptionskoefficient von 800 M⁻¹cm⁻¹ bei einer Wellenlänge von 338 nm der maximalen Absorption) als S-Nitroso-N-(*O*-acetylsalicyl)cysteinethylester identifiziert (C₁₄H₁₆N₂O₆S, MG 340). Die Reinheit der Substanz wird mittels Umkehrphase-Hochdruckflüssigkeitschromatographie (HPLC, Detektion bei 338 nm sowie bei 254 nm) zu >99 % bestimmt.

### C. Alternative Herstellung von S-Nitroso-N-acetylcysteinethylester und S-Nitroso-N-(O-acetylsalicyl)cysteinethylester aus GSNO und NACET bzw. ASS-CET durch S-Transnitrosylierung in einem Zweiphasensystem

Es werden Lösungen von NACET (9.55 mg, 50 µmol) bzw. ASS-CET (15.6 mg, 50 µmol) in eiskaltem Methylenchlorid (je 2 ml) hergestellt. Es werden zwei Lösungen von S-Nitroso-Lglutathion 0.5 H₂O (GSNO; je 20.6 mg je 60 µmol ) in eiskaltem deoxygeniertem Wasser (je 1 ml) hergestellt. Die NACET- und ASS-CET-Lösung werden mit den GSNO-Lösungen für 5 min mit Hilfe eines Vortex-Mixers intensiv gemischt. Dabei entfärben sich die rötlichen wässrigen Phasen, während die organischen Phasen sich rötlich färben. Das ist ein Hinweis für die Umwandlung von GSNO in SNACET bzw. ASS-SNCET. Methylenchlorid wird unter Stickstoff vollständig eingeengt. Es werden jeweils tiefrote ölige Flüssigkeiten erhalten, die nach Aufbewahrung bei -20°C rötliche Kristalle bilden. Die Reinheit der Substanzen wird mittels Umkehrphase-Hochdruckflüssigkeitschromatographie (HPLC, Detektion bei 338 nm) zu je >99 % bestimmt; laut HPLC war kein GSNO in SNACET bzw. ASS-SNCET vorhanden.

Analog erfolgt auch die Herstellung der entsprechenden S-Nitro-Derivaten aus S-Nitroglutathion.

### BEISPIEL 2

### A. Relaxation des humanen Corpus Cavernosum in vitro durch S-Nitroso-N-acetylcysteinethylester, S-Nitroso-N-(O-acetylsalicyl)cysteinethylester und ihre Metabolite.

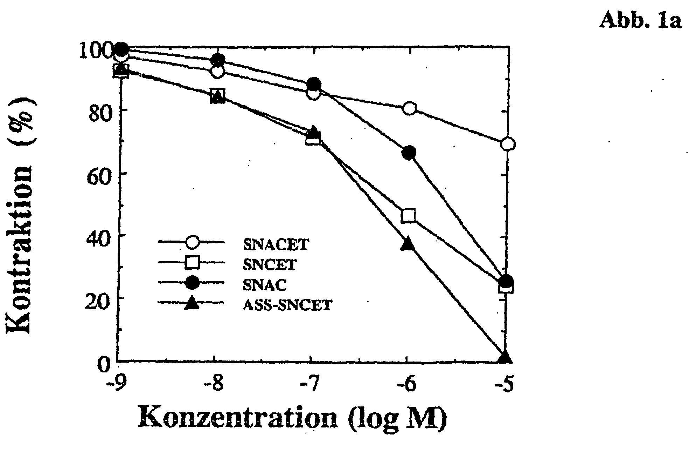

S-Nitroso-N-acetylcysteinethylester (SNACET), S-Nitroso-N-(O-acetylsalicyl)cysteinethylester (ASS-SNCET) und ihre Metabolite S-Nitroso-N-acetylcystein (SNAC) und S-Nitroso-cysteineethylester (SNCET) relaxieren humanes Corpus Cavernosum in vitro in einer Konzentrations-abhängigen Weise (Abb. 1a).

### B. Relaxation der humanen arteria mammaria in vitro durch S-Nitroso-N-acetylcysteinethylester und S-Nitro-N-acetylcysteinethylester.

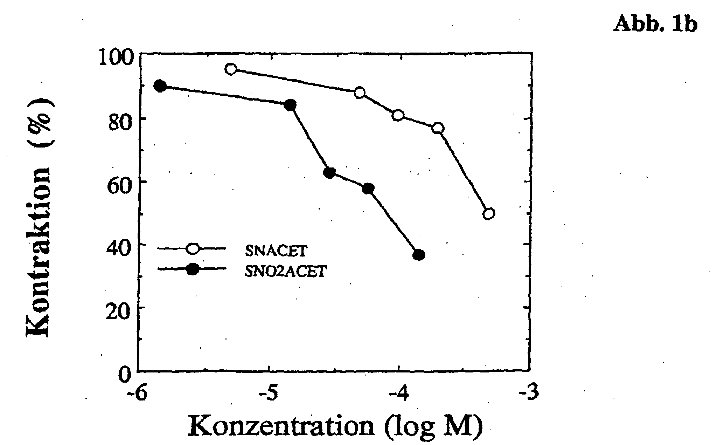

S-Nitroso-N-acetylcysteinethylester (SNACET) und S-Nitro-N-acetylcysteinethylester (SNO₂-ACET) relaxieren humane arteria mammaria in vitro in einer Konzentrations-abhängigen Weise (Abb. 1b).

Die relaxierende Wirkung von SNACE-Derivaten auf das humane Corpus Cavernosum und die humane arteria mammaria zeigt, daß SNACE-Derivate enthaltende Arzneimittel zur Bekämpfung der Impotentia coeundi und von Herzdurchblutungsstörungen als pharmakologische Wirksubstanzen geeignet sind.

### BEISPIEL 3

### Penetration von S-Nitroso-N-acetylcysteinethylester (SNACET) durch humane Tunica albuginea in vitro.

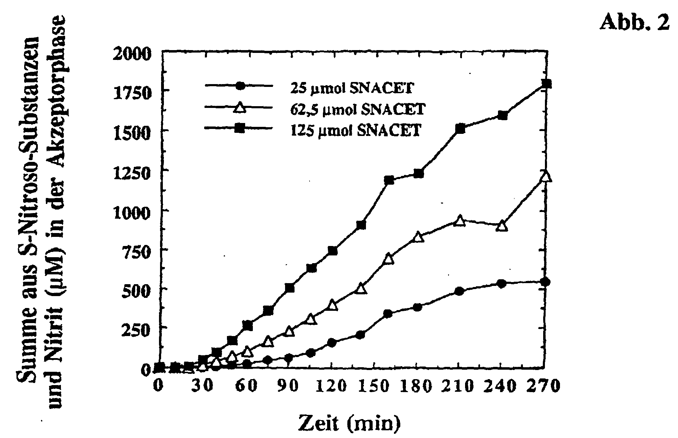

Ein Stück einer frisch präparierten humanen Tunica Albuginea wird in einer Franz' Zelle fixiert. Die aktive Oberfläche beträgt 1.77 cm². Die Acceptorphase (10 ml) besteht aus Hank's Puffer (pH 7.4), Humanalbumin (30 g/l), Penicillin G (100 Einheiten) und Streptomycin (10 µg), und wird mit einem Magnetrührer bei einer konstanten Rotationsgeschwindigkeit gerührt. Die Penetration wird durch Applikation von Lösungen von SNACET in Dimethylsulfoxid unterschiedlichen Gehaltes (0.25 ml) auf das Hautstück gestartet. Zu verschiedenen Zeitpunkten werden aus der Acceptorphase Proben entnommen und die Konzentration der Summe aus SNACET und seinen Metaboliten wird bestimmt. In der Acceptorphase werden unverändertes SNACET und andere Metabolite einschließlich Nitrit nachgewiesen. Abb. 2 zeigt, daß SNACET die Tunica Albuginea penetriert. Die Penetrationsgeschwindigkeit und die Menge des penetrierten SNACET und seiner Metabolite in der Acceptorphase hängen von der auf die Tunica Albuginea applizierten Menge von SNACET ab. Die lag time beträgt ca. 20 min. Im linearen Bereich betragen die Penetrationsraten 1.0, 2.5 und 5.0 µmol SNACET/Stunde.

Ähnliche Ergebnisse wurden auch mit voller Haut von Brust oder Bauch von gesunden Spendern erzielt.

Die Penetration von SNACE-Derivaten durch Tunica Albuginea und voller Haut verdeutlicht, daß die Applikation von SNACE-Derivate enthaltenden Arzneimitteln auf die Haut zur Resorption des Arzneimittels über die Haut führt, wodurch die SNACE-Derivate und ihre Metabolite Zielorgane erreichen und dort therapeutisch wirksam werden.

### BEISPIEL 4

### Hypotensive Wirkung von SNACET in vivo in der Ratte.

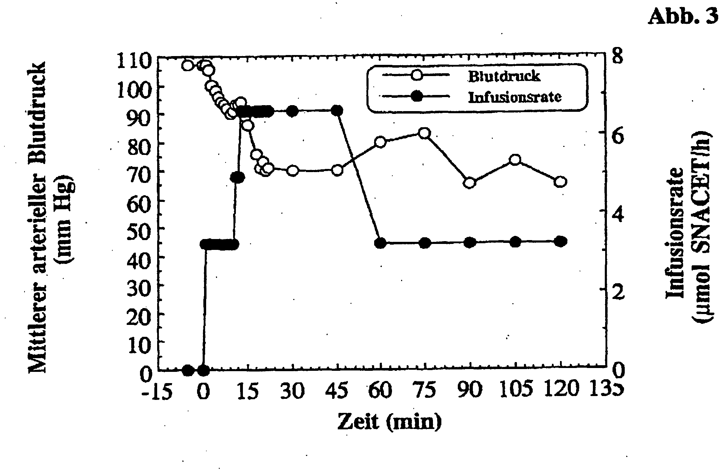

Einer 400 g wiegenden männlichen, anästhesierten Sprague Dawley Ratte wird S-Nitroso-N-acetylcysteinethylester (SNACET) i.v. appliziert. Vor und nach Beginn der Infusion wird der mittlere arterielle Blutdruck blutig kontinuierlich aufgenommen. Abb. 3 zeigt, daß unmittelbar nach Beginn der Infusion von SNACET der mittlere arterielle Blutdruck in Abhängigkeit von der Infusionsrate abnimmt.

### BEISPIEL 5

### Inhibition der Aggregation von humanen Blutplättchen in vitro durch S-Nitroso-N-acetylcysteinethylester (SNACET) und S-Nitroso-N-(O-acetylsalicyl)cysteinethylester (ASS-SNCET).

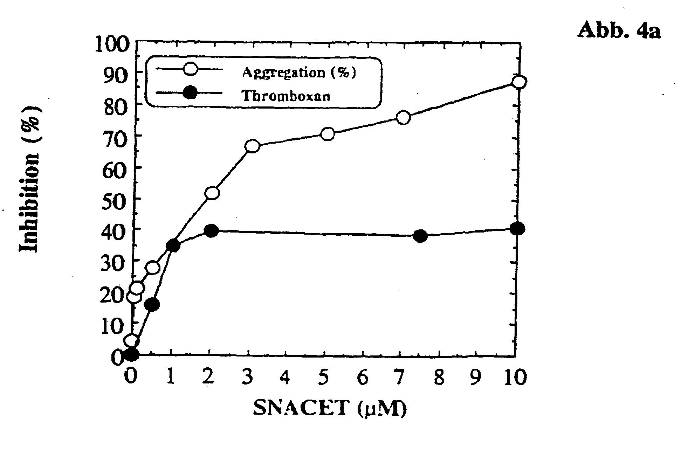

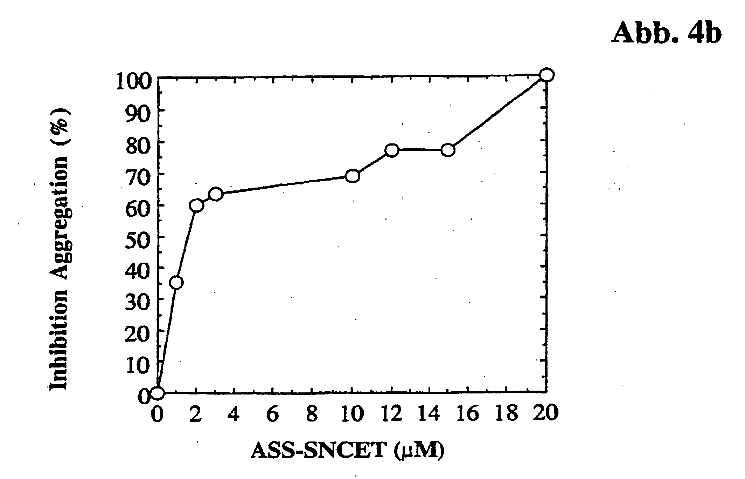

Kollagen-induzierte (2 µg/ml) Aggregation und Thromboxanesynthese in humanen gewaschenen Blutplättchen werden durch SNACET in einer konzentrationsabhängigen Weise inhibiert (Abb. 4a). Die IC₅₀s für die Inhibition der Plättchenaggregation und der Thromboxanesynthese durch SNACET werden zu 1 bzw. 0.5 µM bestimmt. Kollagen-induzierte (2 µg/ml) Aggregation von humanen gewaschenen Blutplättchen wird durch ASS-SNCET in einer konzentrationsabhängigen Weise inhibiert (Abb. 4b). Die IC₅₀ für die Inhibition der Plättchenaggregation durch ASS-SNCET wird zu 2 µM bestimmt (Abb. 4b).

### BEISPIEL 6

### In vivo Metabolismus von oral appliziertem, ¹⁵N-markiertem S-Nitroso-N-acetylcysteinethylester (¹⁵N-SNACET) in der Ratte.

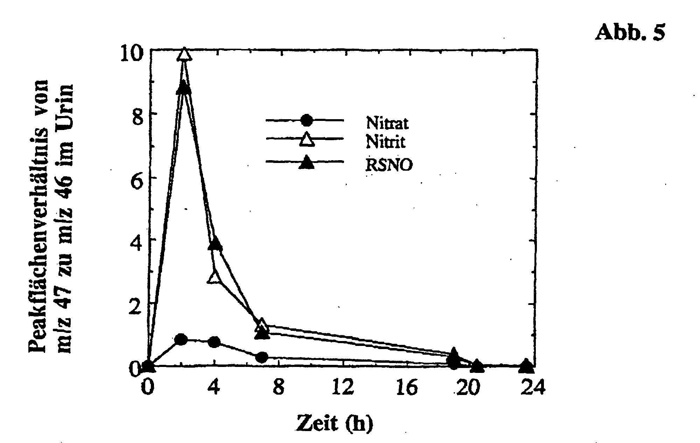

Einer 480 g wiegenden männlichen Sprague Dawley Ratte wird oral 17.5 mg von ¹⁵N-markiertem S-Nitroso-N-acetylcysteinethylester (¹⁵N-SNACET) pro kg Körpergewicht, gelöst in 1 ml einer 10 Gew% Glucose-Lösung in Trinkwasser, verabreicht. Vor und bis zu 24 Stunden nach Applikation wird Urin gesammelt und darin die ¹⁵N-Anreicherung von Nitrat, Nitrit and S-Nitroso-Metaboliten von ¹⁵N-SNACET mittels GC-MS bestimmt. Abb. 5 zeigt daß weder ¹⁵N-SNACET noch andere S-[¹⁵N]Nitroso-haltige Metabolite in den Urin ausgeschieden werden. Als Hauptmetabolit von ¹⁵N-SNACET wird ¹⁵N-Nitrat identifiziert. ¹⁵N-Nitrit wird ebenfalls in den Urin ausgeschieden. Die höchste ¹⁵N-Anreicherung wird in der Urinprobe gemessen, die innerhalb der ersten zwei Stunden gesammelt wird. Die ¹⁵N-Anreicherung im Urin 24 Stunden nach der Gabe von ¹⁵N-SNACET ist geringfügig größer als die natürliche Häufigkeit von ¹⁵N.

Die Ausscheidung von Nitrat im Urin der Ratte nach oraler Applikation von SNACET verdeutlicht, daß oral verabreichte SNACE-Derivate enthaltende Arzneimittel schnell resorbiert und metabolisiert als Nitrat über den Urin ausgeschieden werden.

### ANHANG

### Abbildung 1

a. Relaxation des humanen Corpus Cavernosum in vitro durch S-Nitroso-N-acetylcysteinethylester (SNACET), S-Nitroso-N-(O-acetylsalicyl)cysteinethylester (ASS-SNCET) und ihre Metabolite SNCET und SNAC.
b. Relaxation der humanen arteria mammaria in vitro durch S-Nitroso-N-acetylcysteinethylester (SNACET) und S-Nitro-N-acetylcysteinethylester (SNO₂ACET)

### Abbildung 2

Penetration von S-Nitroso-N-acetylcysteinethylester (SNACET) durch humane Tunica albuginea in vitro in einer Franz Zelle. Drei verschiedene Mengen gelöst in Dimethylsulfoxid (jeweils 250 µl) wurden appliziert (s. Legende).

### Abbildung 3

Hypotensive Wirkung von SNACET in vivo in der Ratte. SNACET wurde i.v. bei den angegebenen Infusionsraten appliziert.

### Abbildung 4

a. Inhibition der Kollagen-induzierten (2 µg/ml) Aggregation von humanen gewaschenen Blutplättchen und der Thromboxansynthese in den Plattchen in vitro durch S-Nitroso-N-acetylcysteinethylester (SNACET).
b. Inhibition der Kollagen-induzierten (2 µg/ml) Aggregation von humanen gewaschenen Blutplättchen in vitro durch S-Nitroso-N-(O-acetylsalicyl)cysteinethylester (ASS-SNCET). Die Werte sind Mittelwerte von Untersuchungen mit Plättchen von vier gesunden Probanden.

### Abbildung 5

Metabolismus von oral appliziertem ¹⁵N-markiertem S-Nitroso-N-acetylcysteinethylester (17.5 mg/kg Körpergewicht) in einer Ratte. ¹⁵N-Anreicherung von Nitrat, Nitrit und für die Summe aus ¹⁵N-markiertem S-Nitroso-N-acetylcysteinethylester und seinen Metaboliten im Urin gesammelt vor und bis zu 24 Stunden nach der Applikation.

## Patentansprüche

1. SNACE-Derivate der allgemeinen Formel I 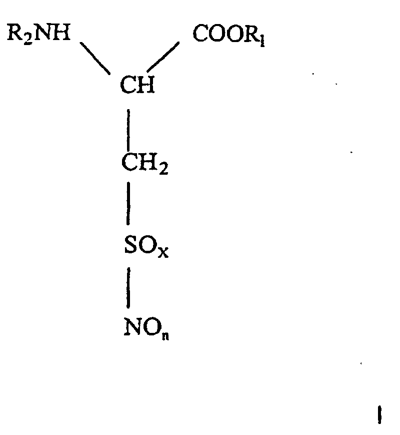 in der n = 1 oder 2 ist, x = 0, 1 oder 2 ist, R₁ ein Alkylrest (C₁₋₂₂) ist und R₂ ein Acylrest (C₂₋₂₂) oder *O*-Acetylsalicylrest ist, mit Ausnahme von S-Nitroso-N-acetyl-Cysteinmethylester.

2. SNACE-Derivate der allgemeinen Formel I, wobei R1 eine Ethylgruppe ist und R2 eine Acetylgruppe ist und n = 1 und x = 0 ist.

3. SNACE-Derivate der allgemeinen Formel I, wobei R1 eine Ethylgruppe ist, R2 eine *O*-Acetylsalicylylgruppe ist und n = 1 und x = 0 ist.

4. Arzneimittel enthaltend Verbindungen der allgemeinen Formel 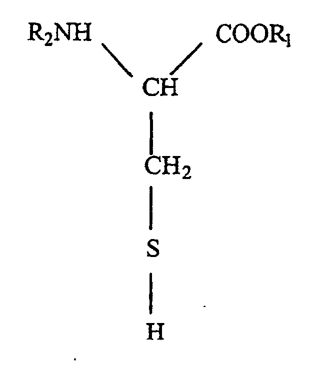 und S-Nitrosoglutathion (GSNO) oder S-Nitroglutathion (GSNO₂) als Prodrug-Komplexe, aus denen die SNACE-Derivate der allgemeinen Formel I (mit x = 0) in situ generiert werden.

5. Arzneimittel enthaltend ein SNACE-Derivat nach einem der Ansprüche 1-4.

6. Verwendung einer Verbindung nach einem der Ansprüche 1-4 zur Herstellung eines Arzneimittels zur Prävention und Behandlung von Durchblutungsstörungen, zur Bekämpfung der Impotentia coeundi, zur Hemmung der Plättchenaggregation, zur Bekämpfung des Asthma bronchiale, zur Behandlung von Mukoviszidose, zur Behandlung der Homocysteinämie, zur Behandlung von Krebs, zur Behandlung der Alzheimer-Krankheit, zur Lipidsenkung, zur Verwendung zur Prävention und zur Behandlung arteriosklerotischer Gefäßveränderungen (Angina pectoris, Herzinfarkt, Schlanganfall, periphere Durchblutungsstörungen), zur Behandlung der Hypertonie und pulmonalen Hypertonie oder zur Behandlung von Abstoßungsreaktionen bei Transplantationen.

7. Arzneimittel gemäß Anspruch 5 zur intravenösen und intraarteriellen Applikation.

8. Arzneimittel nach Anspruch 4 oder 5 zur transkutanen, oralen, rektalen und inhalativen Applikation.

9. Arzneimittel gemäß Anspruch 4 oder 5 in Form von Liposomen, Mikrokapseln oder einer Mikroemulsion oder als transdermales therapeutisches System (TTS).

10. Verwendung nach Anspruch 6, wobei das Arzneimittel zur transkutanen, oralen, rektalen oder inhalativen Applikation ist.

11. Verwendung nach Anspruch 6, wobei das Arzneimittel in Form von Liposomen, Mikrokapseln, einer Mikroemulsion oder als transdermales therapeutisches System (TTS) vorliegt.

## Claims

1. SNACE derivatives of the general formula I 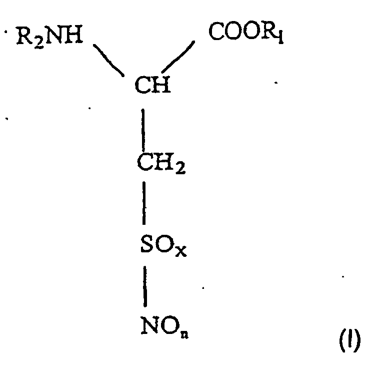 wherein n = 1 or 2, x = 0, 1 or 2, R₁ is (C₁₋₂₂) alkyl and R₂ is (C₂₋₂₂) acyl or *O*-acetylsalicylic, with the exception of S-nitroso-N-acetyl-cysteine methyl ester.

2. SNACE derivatives of the general formula I, wherein R₁ is ethyl and R₂ is acetyl and n = 1 and x = 0.

3. SNACE derivatives of the general formula I, wherein R₁ is ethyl, R₂ is *O*-acetylsalicylyl and n = 1 and x = 0.

4. Pharmaceutical composition containing compounds of the general formula 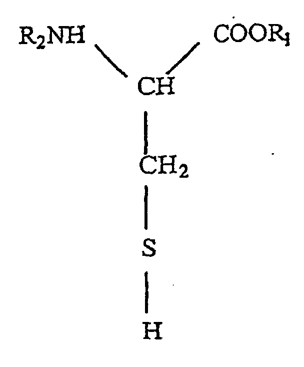 and S-nitrosoglutathione (GSNO) or S-nitroglutathione (GSNO₂) as prodrug complexes from which the SNACE derivatives of the general formula I (with x = 0) are generated in situ.

5. Pharmaceutical composition containing a SNACE derivative according to any one of claims 1 to 4.

6. Use of a compound according to any one of claims 1 to 4 for the production of a pharmaceutical composition for the prevention and treatment of blood flow disorders, for the control of impotentia coeundi, for the inhibition of platelet aggregation, for the control of asthma bronchiale, for the treatment of cystic fibrosis, for the treatment of homocysteinaemia, for the treatment of cancer, for the treatment of Alzheimer's Disease, for lipid reduction, for use in the prevention and treatment of vascular arteriosclerotic disorders (angina pectoris, heart attack, stroke, peripheral blood flow disorders), for the treatment of hypertonia and pulmonary hypertonia or for the treatment of rejection reactions in the case of transplantations.

7. Pharmaceutical composition according to claim 5 for intravenous and intraarterial application.

8. Pharmaceutical composition according to claim 4 or 5 for transcutaneous, oral, rectal or inhalational application.

9. Pharmaceutical composition according to claim 4 or 5 in the form of liposomes, microcapsules or a micro-emulsion or as transdermal therapeutic system (TTS).

10. Use according to claim 6 wherein the pharmaceutical composition is for transcutaneous, oral, rectal or inhalational application.

11. Use according to claim 6 wherein the pharmaceutical composition is present in the form of liposomes, microcapsules, a micro-emulsion or as transdermal therapeutic system (TTS).

## Revendications

1. Dérivé SNACE de formule générale (I) 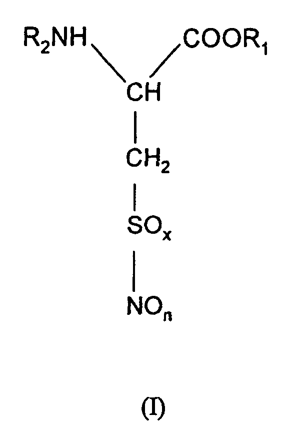 dans laquelle n est 1 ou 2 ; x est 0, 1 ou 2 ; R₁ est un reste (C₁-C₂₂) alkyle et R₂ est un reste (C₂-C₂₂) acyle ou un reste *O*-acétylsalicylique, à l'exception de l'ester méthylique de la S-Nitroso-N-acétyl cystéine.

2. Dérivé SNACE de formule générale (I), dans laquelle R₁ est un groupe éthyle, R₂ est un groupe acétyle, n = 1 et x = 0.

3. Dérivé SNACE de formule générale (I), dans laquelle R₁ est un groupe éthyle, R₂ est un groupe *O*-acétylsalicylique et n = 1 et x = 0.

4. Médicament comprenant des composés de formule générale 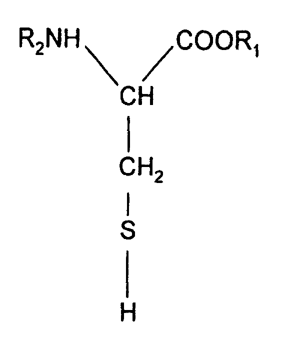 et du S-Nitrosoglutathion (GSNO) ou du S-Nitroglutathion (GSNO2) en tant que complexes pro-drogue, à partir desquels les dérivés SNACE de formule générale I (avec x = 0) sont générés in situ.

5. Médicament comprenant un dérivé SNACE selon l'une des revendications 1-4.

6. Utilisation d'un composé selon l'une des revendications 1-4 pour la fabrication d'un médicament pour la prévention et le traitement de troubles circulatoires, pour lutter conter l'impotence de coeundi, pour inhiber l'agrégation plaquettaire, pour lutter contre les bronchites asthmatiques, pour traiter la mucoviscidose, pour traiter l'hémocystéinémie, pour traiter le cancer, pour traiter la maladie d'Alzheimer, pour diminuer les lipides, pour une utilisation pour la prévention et le traitement de modifications tissulaires artériosclérotiques (angine de poitrine, infarctus du myocarde, accident vasculaire cérébral, troubles circulatoires périphériques), pour traiter l'hypertonie et l'hypertonie pulmonaire, ou pour le traitement des réactions de rejet lors de transplantations.

7. Médicament selon la revendication 5 pour application intraveineuse et intra-artérielle.

8. Médicament selon la revendication 4 ou 5 pour application transcutanée, orale, rectale et par inhalation.

9. Médicament selon la revendication 4 ou 5 sous forme de liposomes, microcapsules ou d'une microémulsion ou d'un système thérapeutique transdermique (STT).

10. Utilisation selon la revendication 6, selon laquelle le médicament est appliqué de manière transcutanée, orale, rectale ou par inhalation.

11. Utilisation selon la revendication 6, selon laquelle le médicament est présenté sous forme de liposomes, microcapsules ou d'une microémulsion ou d'un système thérapeutique transdermique (STT).
